(19) Europäisches Patentamt
European Patent Office
Office européen des brevets

(11) EP 0 581 954 B1

(12) # EUROPÄISCHE PATENTSCHRIFT

(45) Veröffentlichungstag und Bekanntmachung des Hinweises auf die Patenterteilung:
**24.07.1996 Patentblatt 1996/30**

(21) Anmeldenummer: **93917369.6**

(22) Anmeldetag: **03.02.1993**

(51) Int Cl.[6]: **C07C 49/835**, C07C 49/83, C07C 69/738, C07C 49/747, A61K 7/42, A61K 31/12, C07D 235/18, C07D 251/26

(86) Internationale Anmeldenummer:
**PCT/EP93/00245**

(87) Internationale Veröffentlichungsnummer:
**WO 93/16026 (19.08.1993 Gazette 1993/20)**

(54) **VERWENDUNG VON ALPHA-HYDROXYKETOALKYL-DERIVATEN ALS LICHTSCHUTZFILTER**

USE OF ALPHA-HYDROXYKETOALKYL DERIVATIVES AS PROTECTIVE LIGHT FILTERS

UTILISATION DE DERIVES ALPHA-HYDROXYCETOALKYLE COMME SUBSTANCES FILTRANTES PROTECTRICES POUR L'EXPOSITION AUX RAYONS LUMINEUX

(84) Benannte Vertragsstaaten:
**DE ES FR GB IT**

(30) Priorität: **15.02.1992 DE 4204651**

(43) Veröffentlichungstag der Anmeldung:
**09.02.1994 Patentblatt 1994/06**

(73) Patentinhaber: **MERCK PATENT GmbH**
**D-64271 Darmstadt (DE)**

(72) Erfinder:
- **MARTIN, Roland**
  **D-6940 Weinheim (DE)**
- **STEIN, Ingeborg**
  **D-6106 Erzhausen (DE)**
- **HEYWANG, Ulrich**
  **D-6100 Darmstadt (DE)**

(56) Entgegenhaltungen:
**FR-A- 2 556 592**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 581 954 B1

## Beschreibung

Die Erfindung betrifft die Verwendung von α-Hydroxyketoalkyl-Derivaten als Lichtschutzfilter für kosmetische oder pharmazeutische Produkte, wobei die α-Hydroxyketoalkylgruppe, gegebenenfalls über einen Spacer, mit einer chromophoren Gruppe, welche in einem Wellenlängenbereich zwischen 280 und 400 nm ein Absorptionsmaximum und ein konjungiertes π-Elektronensystem von mindestens 8 π-Elektronen aufweist, verknüpft ist, sowie neue α-Hydroxyketoalkyl-Derivate, Verfahren zu deren Herstellung und deren Verwendung in kosmetischen Zubereitungen, insbesondere zum Schutz vor Sonnenstrahlung, und in pharmazeutischen Zubereitungen zur vorbeugenden Behandlung von Entzündungen und Allergien der Haut oder bestimmter Krebsarten.

Bekanntlich reagiert die Haut empfindlich auf Sonnenstrahlen, welche einen gewöhnlichen Sonnenbrand oder ein Erythem, aber auch mehr oder weniger ausgeprägte Verbrennungen hervorrufen können.

Sonnenstrahlen haben aber auch andere negative Wirkungen: sie bewirken, daß die Haut ihre Elastizität verliert und sich Falten bilden und führen somit zu einer frühzeitigen Alterung. Manchmal kann man auch Dermatosen beobachten. Im extremen Fall kommt es bei manchen Menschen zum Auftreten von Hautkrebs.

Es ist auch wünschenswert, Haare gegen photochemische Schäden zu schützen, um Veränderungen von Farbnuancen, ein Entfärben oder Schäden mechanischer Art zu verhindern.

Es ist bekannt, daß die in kosmetischen Präparaten enthaltenen Komponenten nicht immer genügend lichtstabil sind und sich unter der Einwirkung von Lichtstrahlen zersetzen.

Bekanntlich wird der gefährlichste Teil der Sonnenstrahlen von den ultravioletten Strahlen mit einer Wellenlänge von weniger als 400 nm gebildet. Bekannt ist auch, daß durch das Vorhandensein der Ozonschicht der Erdatmosphäre, die einen Teil der Sonnenstrahlung absorbiert, die untere Grenze der ultravioletten Strahlen, welche die Erdoberfläche erreichen, bei ca. 280 nm liegt.

Es erscheint somit wünschenswert, Verbindungen zur Verfügung zu stellen, welche UV-Strahlen in einem Wellenlängenbereich von 280 bis 400 nm absorbieren können, d.h. auch UV-B-Strahlen mit einer Wellenlänge zwischen 280 und 320 nm, die bei der Bildung eines Sonnenerythems eine entscheidende Rolle spielen, wie auch UV-A-Strahlen mit einer Wellenlänge zwischen 320 und 400 nm, welche die Haut bräunen aber auch altern lassen, die Auslösung einer erythematösen Reaktion begünstigen oder diese Reaktion bei bestimmten Menschen vergrößern oder sogar phototoxische oder photoallergische Reaktionen auslösen können.

Die heute üblichen Sonnenschutzfilter in der Kosmetik werden in UVA- bzw. UVB-Filter unterteilt. Während es im UVB-Bereich (280-320 nm) mit Substanzen wie Eusolex® 6300 oder Eusolex® 232 gute Filter gibt, sind die im UVA-Bereich (320-400 nm) verwendeten problembehaftet:

Dibenzoylmethane wie Parsol® 1789 oder Eusolex® 8020 sind bei UV-Bestrahlung nicht unbegrenzt stabil, was zum einen die Filtereffektivität mit der Zeit herabsetzt und zum anderen Photosensibilisierungen der Haut in vereinzelten Fällen begünstigen kann. Die ebenfalls als UVA-Filter verwendeten Benzophenone sind in den in der Kosmetik verwandten Ölen nur begrenzt löslich, und sie weisen eine relativ geringe Absorption auf. Dagegen sind nur wenige wasserlösliche UVA-Filter derzeit bekannt, deren UV-Absorption jedoch gering ist.

Aus der EP 03 90 682 sind ähnliche Benzylidenkampfer-Derivate bekannt. Diese weisen jedoch mindestens eine Hydroxygruppe am Phenylenring auf. Diese Verbindungen können zwar auch als UV-Filter in Sonnenschutzmitteln verwendet werden, eignen sich jedoch aufgrund der phenolischen Hydroxylgruppe eher als Antioxidantien. Weiterhin sind diese Verbindungen in herkömmlichen kosmetischen Trägern, insbesondere in wäßrigen Suspensionen, nur begrenzt löslich, so daß sie in Sonnenschutzmitteln meist zusammen mit anderen UV-Filtern eingesetzt werden müssen.

Die herkömmlichen Lichtschutzfilter weisen in der Regel eine geringe oder eine ungenügende Hauthaftung auf, was zu einer kürzeren Dauer der Schutzwirkung des Filters und insbesondere zur nahezu vollständigen Entfernung des Filters beim Baden führt.

Es wurde gefunden, daß bestimmte α-Hydroxyketoalkyl-Derivate insbesondere 4-(1-Oxo-2-hydroxyethyl)phenyl-Derivate hervorragende UVB-Filter-Eigenschaften besitzen. Ihre Löslichkeit in den in der Kosmetik verwandten Ölen ist sehr gut, so daß Einsatzkonzentrationen auch in komplizierten Formulierungen bis mindestens 10 % der Zubereitung möglich sind. Eine wasserlösliche Form des neuen Filters sind die entsprechenden Sulfonsäuren. Die Wasserlöslichkeit ist hier so gut, daß ebenfalls Einsatzkonzentrationen von 10 % möglich sind.

Darüber hinaus verfügen die α-Hydroxyketoalkyl-Derivate über eine vorzügliche Hauthaftung und in einigen Fällen über einen Selbstbräunungseffekt, wie z.B. andere Hydroxyketoverbindungen (Dihydroxyaceton).

Weiterhin weisen die erfindungsmäßigen Verbindungen eine außerordentliche Photostabilität gegenüber UV-Strahlung auf, die die Stabilität bisher bekannter UV-Filtersubstanten bei weitem übersteigt.

Falls die Extinktion im UVA-Bereich ein Minimum aufweist, ist kein Nachteil, da man problemlos einen UVA-Filter mit in die Formulierung einarbeiten kann.

Weiterhin können die Verbindungen der Formel I auch zur vorbeugenden Behandlung von Entzündungen und Allergien der Haut und zur Verhütung bestimmter Krebsarten verwendet werden.

Außer ihren guten Eigenschaften als Filter zeichnen sich die erfindungsgemäßen Verbindungen durch eine gute

thermische und photochemische Stabilität aus.

Ferner bieten diese Verbindungen den Vorteil, nicht toxisch oder reizend und gegenüber der Haut vollkommen unschädlich zu sein.

Sie verteilen sich gleichmäßig in den herkömmlichen kosmetischen Trägern und können insbesondere in Fett-Trägern einen kontinuierlichen Film bilden; sie können auf diese Weise auf die Haut aufgetragen werden, um einen wirksamen Schutzfilm zu bilden.

Gegenstand der Erfindung ist die Verwendung von α-Hydroxyketoalkyl-Derivaten als Lichtschutzfilter für kosmetische Produkte, wobei die α-Hydroxyketoalkylgruppe, gegebenenfalls über einen Spacer, mit einer chromophoren Gruppe, welche in einem Wellenlängenbereich zwischen 280 und 400 nm ein Absorptionsmaximum und ein konjungiertes π-Elektronensystem von mindestens 8 π-Elektronen aufweist, verknüpft ist.

Ein weiterer Gegenstand der Erfindung sind α-Hydroxyketoalkyl-Derivate der Formel I

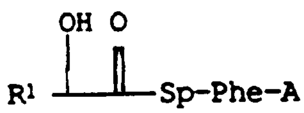

(I)

wobei

- $R^1$ H oder Alkyl mit 1 bis 10 C-Atomen,
- Sp $(CH_2)_n$ oder -CH=CH-,
- n 0 oder eine ganze Zahl zwischen 1 und 10,
- Phe unsubstituiertes oder durch 1 bis 4 Hydroxy-, Alkyl- oder Alkyloxygruppen, worin die Alkylgruppen jeweils 1-10 C-Atome aufweisen, substituiertes Phenylen bedeutet,
- A eine Substituentengruppe mit insgesamt bis zu 60 Kohlenstoff-, Schwefel-, Stickstoff- und Sauerstoffatomen in der Grundstruktur bedeutet, welche ein konjungiertes π-Elektronensystem von mindestens 4 π-Elektronen in Konjugation zur Gruppe Phe aufweist.

Die Substituentengruppe A weist ein 4 π-Elektronensystem auf, das gegebenenfalls in einem aromatischen oder heteroaromatischen Ringsystem eingebunden ist, in der Regel enthält es 1 bis 4 Sauerstoff- oder Stickstoffatome und umfaßt Grundstrukturen, die gemäß dem Stand der Technik UV-Filtereigenschaften hervorrufen oder aufweisen; bevorzugt bedeutet die Substituentengruppe

A einen Rest ausgewählt aus den Formeln (a) bis (g)

(a) - CH=CH-COOR$^2$

(b) 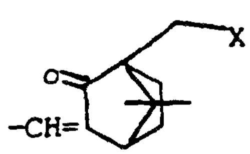

(c) 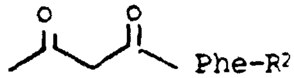

(d) 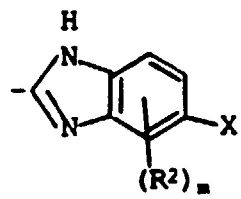

(e) 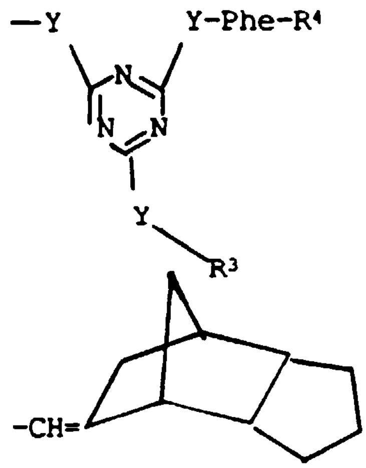

(f)

(g) 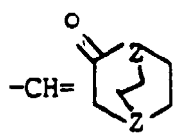

worin jeweils unabhängig voneinander

- $R^2$ eine für $R^1$ angegebene Bedeutung besitzt,
- Phe die angegebene Bedeutung besitzt,
- X H, $R^2$ oder $SO_3H$ bedeutet,
- m 0, 1, 2 oder 3 ist,
- Y jeweils unabhängig voneinander 0 oder NH ist,
- Z jeweils unabhängig voneinander CH oder N,
- $R^3$ eine der Bedeutungen von $R^1$ besitzt oder Phe-$R^4$ bedeutet, worin $R^4$ einen Rest ausgewählt aus den Formeln (a), (b), bedeutet, (d) (f) und (g)

bedeuten.

In diesen Formeln steht $R^1$ und $R^2$ für Wasserstoff, geradkettiges oder verzweigtes Alkyl, vorzugsweise einen Methyl-, Ethyl-, n-Propyl-, i-Propyl-, n-Butyl-, i-Butyl-, t-Butyl-oder 1,1,3,3-Tetramethylbutylrest oder Wasserstoff, insbesondere für Wasserstoff.

Die Phenylengruppe (Phe) ist vorzugsweise eine unsubstituierte oder durch eine bis vier Alkyl- oder Alkoxygruppen substituierte 1,4-Phenylengruppe.

Vorzugsweise ist die Phenylengruppe (Phe) unsubstituiert oder substituiert mit einer oder zwei Alkoxygruppen mit

1 bis 8 C-Atomen, insbesondere mit Methoxy, Ethoxy- oder 2-Ethyl-hexyloxygruppen.

Bevorzugte Verbindungen der Formel I sind diejenigen der Formeln I1 bis I8, wobei A die angegebene Bedeutung besitzt und

$R^7$ Alkyl oder Alkoxy mit 1 bis 10 C-Atomen bedeutet und n 1 oder 2 bedeutet.

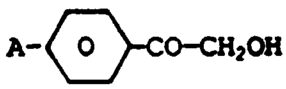

I1

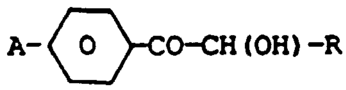

I2

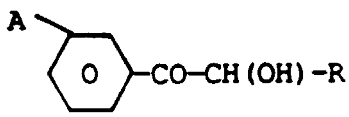

I3

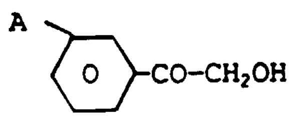

I4

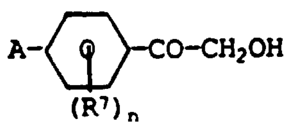

I5

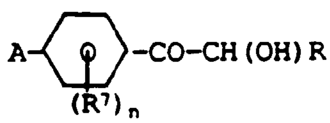

I6

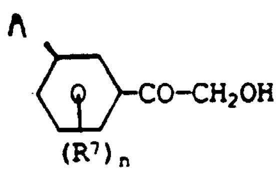

I7

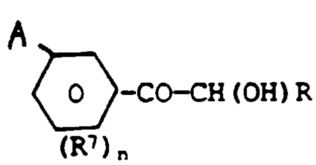

I8

Weitere bevorzugte Ausführungsformen sind:

a) α-Hydroxyketoalkyl-Derivate, worin
Phe unsubstituiertes 1,4-Phenylen
bedeutet;

b) α-Hydroxyketoalkyl-Derivate, worin

n 0
bedeutet;
c) α-Hydroxyketoalkyl-Derivate, worin

$R^1$ H

bedeutet;
d) α-Hydroxyketoalkyl-Derivate der Formel Ia

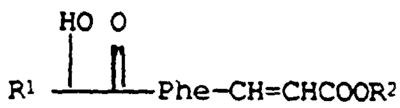

(Ia);

e) α-Hydroxyketoalkyl-Derivate der Formel Ib

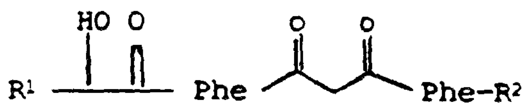

(Ib);

f) α-Hydroxyketoalkyl-Derivate der Formel Ic

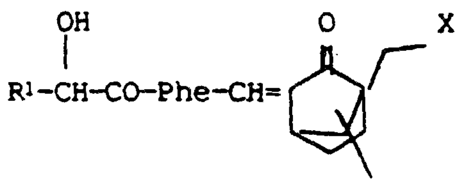

(Ic)

und
g) α-Hydroxyketoalkyl-Derivate, worin Phe eine unsubstituierte 1,4-Phenylengruppe und $R^1$ H bedeutet.

Man erhält die Verbindungen der Formel I z.B. dadurch, daß man ein Formylbenzyliden-Derivat der Formel II,

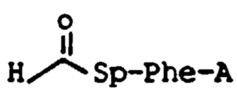

(II)

worin Phe, Sp und A die angegebene Bedeutung besitzen, in Gegenwart einer Base und einer katalytischen Menge eines Thiazoliumhalogenids mit einem Aldehyd der Formel $R^1$-CHO umsetzt, oder daß man das Derivat der Formel II in ein Derivat der Formel III überführt

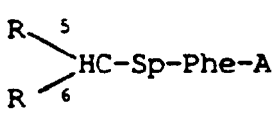

(III)

wobei

| | |
|---|---|
| $R^5$ | CN und |
| $R^6$ | NR'R" oder OR"', worin R' und R" jeweils unabhängig voneinander Alkyl mit 1-6 C-Atomen oder Phenyl und R"' Si $(CH_3)_3$, oder |
| $R^5$ und $R^6$ | jeweils unabhängig voneinander SR', oder zusammengenommen $S(CH_2)_nS$ mit n 2, 3 oder 4 |

bedeuten,
mit einer starken Base behandelt und mit einem Aldehyd der Formel $R^1$-CHO umsetzt .

Die Methode I beinhaltet die katalytische "Umpolung" mit einem Thiazoliumhalogenid in Gegenwart einer Base.

Vorzugsweise verwendet man 3-Alkylbenzothiazoliumhalogenide, insbesondere 3-Ethylbenzothiazoliumbromid.

Die Reaktion wird, in der Regel, in einem Verdünnungsmittel, vorzugsweise einem protischen Lösungsmittel, insbesondere einem Alkohol, wie z.B. Methanol, Ethanol oder Isopropanol, durchgeführt. Als Base setzt man vorzugsweise schwache Basen, insbesondere primäre, sekundäre oder tertiäre Amine, wie z.B. Triethylamin, Imidazol oder Pyridin ein.

Die Reaktion kann bei Temperaturen zwischen 0 °C und dem Siedepunkt des Reaktionsgemisches durchgeführt werden, vorzugsweise arbeitet man bei Raumtemperatur.

Die Methode II beinhaltet die Überführung der Formyl-Derivate der Formel II in ein Derivat der Formel III, welches ein acides, benzylisches Wasserstoffatom aufweist.

Bevorzugte Verbindungen der Formel III sind diejenigen, worin $R^1$ und $R^2$ zusammengenommen $S(CH_2)_nS$ mit n 2 oder 3 bedeuten, oder worin $R^5$ CN und $R^6$ $OSi(CH_3)_3$ o der NR'R" bedeutet.

Diese Verbindung enthält man in an sich bekannter Weise durch Umsetzen des Formyl-Derivates der Formel III

a) mit einem Alkandithiol unter Wasser abspaltenden Bedingungen,

b) mit Trimethylsilylcyanid oder

c) mit einer sekundären Amin und einer Alkalicyamid.

Die Deprotonierung gelingt in der Regel mit einer starken Base, vorzugsweise mit n-Butylithium oder Lithiumdiisopropylamid.

Das erhaltene Addukt der Formel IV

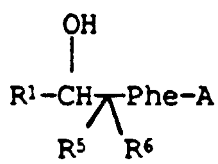

(IV)

wird nach sich bekannter Methode, d. h. mit verdünnter Säure, gegebenenfalls in Gegenwart von Schwermetallsalzen, wie z.B. Quecksilber, Kupfer oder Silbersalzen, hydrolysiert.

Die Aldehyde der Formel II sind bekannt (z.B. DE 28 11 041) oder werden nach bekannten Methoden hergestellt.

Die Derivate der Formel III und IV sind neu und ebenfalls Gegenstand der Erfindung.

Man erhält die Verbindungen der Formel I, worin A einen Rest der Formel (e) bedeutet, z.B. dadurch, daß man ein α-Hydroxy-ketoalkylphenol bzw. -anilin-Derivat der Formel V,

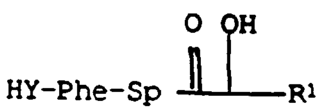

(V)

worin Phe, $R^1$, Sp und Y die angegebene Bedeutung besitzen, mit Cyanurchlorid umsetzt.

Wird die Reaktion bei niedrigen Temperaturen, d.h. zwischen -40 °C und 80 °C, vorzugsweise 0 °C und 60 °C, insbesondere zwischen 0 °C und Raumtemperatur, gegebenenfalls in Gegenwart einer schwachen Base, mit nur ca. 1 Mol an Verbindung der Formel II, bezogen auf 1 Mol Cyanurchlorid, durchgeführt, erhält man intermediär ein Triazin-Derivat der Formel VI,

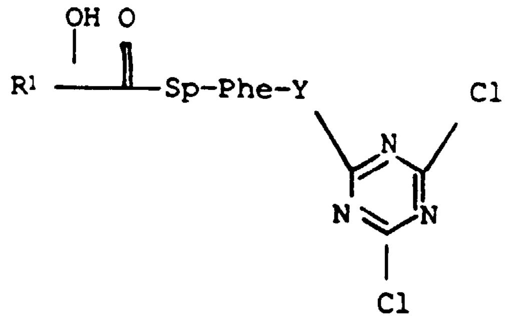

(VI)

worin $R^1$, Sp, Phe und Y die angegebene Bedeutung besitzen.

Anschließend wird die Verbindung der Formel VI mit einem oder zwei weiteren Equivalent(en) einer Verbindung der Formel $R^4$-Phe-Y, worin Y, Phe und $R^4$ die angegebene Bedeutung besitzt, von der ursprünglich eingesetzten und gegebenenfalls anschließend mit einem Alkohol, Alkylamin, Anilin oder Phenol mit 1 bis 10 C-Atomen umgesetzt.

Bei Umsetzung von ca. 2 Mol an Verbindung der Formel $R^4$-Phe-YH mit Cyanurchlorid bei diesen Temperaturen erhält man intermediär ein Triazinderivat der Formel VII,

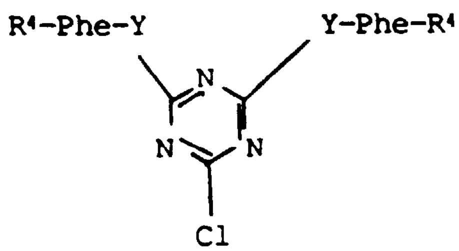

(VII)

worin $R^4$, Phe und Y die angegebene Bedeutung besitzen, woraus man durch Umsetzung mit ca. 1 Mol an Verbindung der Formel V eine Verbindung der Formel I (e) herstellt.

Die Verbindungen der Formel V und VI sind neu und ebenfalls Gegenstand der Erfindung.

Die Herstellung der neuen Verbindungen der Formel V erfolgt in Analogie zur Herstellung der Verbindungen der Formel I nach Methode 1 oder 2 durch "Umpolung" der entsprechenden Hydroxy- bzw. Aminobenzaldehyde der Formel

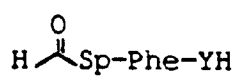

und Umsetzung mit einem Aldehyd der Formel $R^1$-CHO.

Man erhält die Verbindungen der Formel I, worin A einen Rest der Formel (c) darstellt, vorzugsweise durch eine Claisen-Kondensation eines Esters der Formel VIII

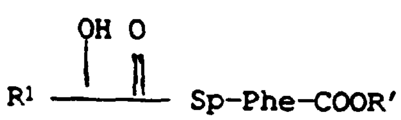

(VIII)

mit einem Acetophenonderivat der Formel IX

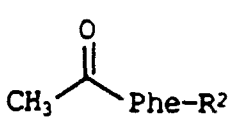

(IX)

z.B. nach B.R. Hauser et al. Organic Reactions Vo. VIII, S. 5 %, John Wiley u. Sons Inc. New York 1954.

Die Verbindungen der Formel VIII sind ebenfalls neu und können in Analogie zur Methode 1 oder 2 aus Formylbenzoesäureestern hergestellt werden.

Ein weiterer Gegenstand der Erfindung ist eine kosmetische Zubereitung, welche in einem kosmetisch verträglichen Träger eine wirksame Menge mindestens eines Derivates der obigen Formel I enthält.

Insbesondere bevorzugt sind solche kosmetischen Zubereitungen, worin der Träger mindestens eine Fettphase aufweist und in der Verbindung der Formel I X H bedeutet, bzw. solche, worin der Träger mindestens eine wäßrige Phase aufweist und X $SO_3H$ bedeutet.

Das erfindungsgemäße kosmetische Mittel kann als Mittel zum Schutz der menschlichen Epidermis oder der Haare oder als Sonnenschutzmittel verwendet werden.

Gegenstand der Erfindung ist ferner ein Verfahren zum.Schutz der Haut und natürlicher oder sensibilisierter Haare von Sonnenstrahlen, wobei auf die Haut oder die Haare eine wirksame Menge mindestens einer Verbindung der Formel I aufgetragen wird.

Mit "sensibilisierten Haaren" sind Haare gemeint, welche einer Dauerwellenbehandlung, einem Färbe- oder Entfärbeprozeß unterzogen worden sind.

Gegenstand der Erfindung ist ferner eine gefärbte oder ungefärbte lichtstabilisierte kosmetische Zubereitung, welche eine wirksame Menge mindestens eines Benzylidenkampfer-Derivates der obigen Formel I umfaßt.

Wird das erfindungsgemäße kosmetische Mittel als Mittel zum Schutz menschlicher Epidermis gegen UV-Strahlen verwendet, so liegt es in verschiedenen, für diesen Typ üblicherweise verwendeten Formen vor. So kann es insbesondere in Form öliger oder ölig-alkoholischer Lotionen, Emulsionen, wie als Creme oder als Milch in Form ölig-alkoholischer, öligwäßriger oder wälirig-alkoholischer Gele oder als feste Stifte vorliegen oder als Aerosol konfektioniert sein.

Es kann kosmetische Adjuvanzien enthalten, welche in dieser Art von Mittel üblicherweise verwendet werden, wie z.B. Verdickungsmittel, weichmachende Mittel, Befeuchtungsmittel, grenzflächenaktive Mittel, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Die Verbindung der Formel I ist in der Regel in einer Menge von 0,5 bis 10 % bezogen auf das Gesamtgewicht des kosmetischen Mittels zum Schutz menschlicher Epidermis, enthalten.

Man kann als Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsform der Erfindung ist eine Emulsion, welche als Schutzcreme oder -milch vorliegt und außer der Verbindung der Formel I Fettalkohole, Fettsäureester, insbesondere Triglyceride von Fettsäuren, Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycols, wie Propylenglykol, und/oder eines Polyols, wie Glycerin, und Ölen, Wachsen und Fettsäureestern, wie Triglyceriden von Fettsäuren, dar.

Das erfindungsgemäße kosmetische Mittel kann auch als alkoholisches Gel vorliegen, welches einen oder mehrere Niedrigalkohole oder -polyole, wie Ethanol, Propylenglycol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäureestern, Lanolin und anderen Fettkörpern.

Gegenstand der Erfindung sind auch kosmetische Sonnenschutzmittel, die mindestens eine Verbindung der Formel I enthalten und andere UVB- und/oder UVA-Filter umfassen können.

In diesem Fall beträgt die Menge des Filters der Formel I in der Regel zwischen 1,0 und 8,0 Gew.% bezogen auf das Gesamtgewicht des Sonnenschutzmittels.

Ist ein Mittel als Aerosol konfektioniert, verwendet man in der Regel die üblichen Treibmittel, wie Alkane, Fluoralkane und Chlorfluoralkane.

Soll das erfindungsgemäße Mittel natürliche oder sensibilisierte Haare vor Uv-Strahlen schützen, so kann es als Shampoo, Lotion, Gel oder Emulsion zum Ausspülen vorliegen, wobei die jeweilige Formulierung vor oder nach dem Shamponieren, vor oder nach dem Färben oder Entfärben, vor oder nach der Dauerwelle aufgetragen wird; oder das Mittel liegt als Lotion oder Gel zum Frisieren und Behandeln, als Lotion oder Gel zum Bürsten oder Legen einer Wasserwelle, als Haarlack, Dauerwellmittel, Färbe- oder Entfärbemittel der Haare vor. Dieses Mittel kann außer der erfindungagemäßen Verbindung verschiedene, in diesem Mitteltyp verwendete Adjuvantien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien. Das Mittel enthält in der Regel 1,0 bis 5,0 Gew.% der Verbindung der Formel I.

Die vorliegende Erfindung befaßt sich auch mit kosmetischen Mitteln, die mindestens eine Verbindung der Formel I als Mittel zum Schutz vor UV-Strahlen und als Antioxidationsmittel enthalten; diese Mittel umfassen Haarprodukte, wie Haarlacke, Wasserwell-Lotionen zum Einlegen der Haare, gegebenenfalls zum Behandeln oder leichteren Frisieren, Shampoos, Färbeshampoos, Haarfärbemittel, Schminkprodukte, wie Nagellack, Cremes und Öle zur Hautbehandlung, Make-up (Fond de teint), Lippenstifte, Hautpflegemittel, wie Badeöle oder -cremes und andere kosmetische Mittel, die im Hinblick auf ihre Komponenten Probleme mit der Lichtstabilität und/oder der Oxidation im Laufe des Lagerns aufwerfen können. Derartige Mittel enthalten in der Regel 1,0 bis 5,0 Gew.% einer Verbindung der Formel I.

Ferner befaßt sich die Erfindung mit einem Verfahren zum Schutz der kosmetischen Mittel vor UV-Strahlen und Oxidation, wobei diesen Mitteln eine wirksame Menge mindestens einer Verbindung der Formel I zugesetzt wird.

Ein weiterer Gegenstand der Erfindung ist die Verwendung der Verbindungen der Formel I als Sonnenfilter von großer Absorptionsbreite in einem Wellenlängenbereich von 320 bis 400 nm.

Ein weiterer Gegenstand der Erfindung ist die Verwendung der Verbindungen der Formel I als kosmetische Produkte.

Wie oben bereits erwähnt, hat die Anmelderin im Laufe ihrer Untersuchungen außerdem festgestellt, daß die Verbindungen der Formel I eine signifikante pharmakologische Aktivität auf dem Gebiet der Präventivbehandlung von Entzündungen und Hautallergien zeigen.

Gegenstand der Erfindung sind auch die Verbindungen der Formel I zur Verwendung als Medikament.

Gegenstand der Erfindung ist ferner ein pharmazeutisches Mittel, welches eine wirksame Menge mindestens einer Verbindung der Formel I als Wirkstoff in einem nicht-toxischen Träger oder Exzipienten enthält.

Das erfindungsgemäße pharmazeutische Mittel kann oral oder topisch verabreicht werden.

Für die orale Verabreichung liegt das pharmazeutische Mittel in Form von Pastillen, Gelatinekapseln, Dragees, als Sirup, Suspension, Lösung, Emulsion etc. vor. Zur topischen Verabreichung liegt es als Salbe, Creme, Pomade, Lösung, Lotion, Gel, Spray, Suspension etc. vor.

Dieses Mittel kann inerte oder pharmakodynamisch aktive Additive enthalten, insbesondere Hydratisierungsmittel, Antibiotika, steroide oder nicht-steroide antiinflammatorische Mittel, Carotinoide und Mittel gegen Psoriasis.

Dieses Mittel kann auch Geschmacksverbesserungsmittel, Konservierungsmittel, Stabilisatoren, Feuchtigkeitsregulatoren, pH-Regulatoren, Modifikatoren für den osmotischen Druck, Emulgatoren, Lokalanaesthetika, Puffer etc. enthalten.

Es kann außerdem in an sich bekannter Weise in Retard-Form oder in einer Form konditioniert sein, in welcher der Wirkstoff rasch freigesetzt wird.

Die nachfolgenden Beispiele sind repräsentativ für die vorliegende Erfindung.

Auch ohne weitere Ausführungen wird davon ausgegangen, daß ein Fachmann die obige Beschreibung in weitesten Umfang nutzen kann. Die bevorzugten Ausführungsformen sind deswegen lediglich als beschreibende, keineswegs als in irgendeine Weise limitierende Offenbarung aufzufasssen.

**Beispiel 1**

3-(4'-(2"-Hydroxy-1"-oxoethyl)-benzyliden)-campher

Zu einer Reaktionsmischung von 10 g (35 mMol) 3-(4'-Formyl-benzyliden)-campher (hergestellt z.B. nach DE 2811041) und 1,2 g Paraformaldehyd in 70 ml Ethanol werden 0,8 g (3,5 mMol) 3-Ethyl-benzothiazolium-bromid und Triethylamin zugegeben und unter Rückfluß erhitzt. Nach beendeter Reaktion wird eingeengt, der Rückstand in Essigester aufgenommen und mit Wasser gewaschen. Die Aufreinigung erfolgt mittels Säulenchromatografie.

Es entstehen gelbe Kristalle mit dem Schmelzpunkt von 98 °C. Die Spektren entsprechen der erwarteten Verbindung. UV (Ethanol, c = 1 mg/100 ml): $\lambda_{max}$ = 305, E = 1 (d = 1 cm) Löslichkeit: 5 % in Miglyol

Analog werden hergestellt:

3-(3'-(2"-Hydroxy-1"-oxoethyl)-benzyliden)-campher
3-(4'-(2"-Hydroxy-1-oxoethyl)-2',5'-dimethoxybenzyliden)-campher
3-(4'-(2"-Hydroxy-1"-oxopropyl)-benzyliden)-campher
3-(3'-(2"-Hydroxy-1"-oxopropyl)-benzyliden)-campher
3-(4'-(2"-Hydroxy-1"-oxobutyl)-benzyliden)-campher
3-(4'-(2"-Hydroxy-1"-oxopentyl)-benzyliden)-campher

**Beispiel 2**

**<u>2A</u> <u>4-(Propenylcarbonsäureethylester)-benzaldehyddiethyl-acetal</u>**

0,5 Mol Essigsäureethylester werden bei 0 °C zu 0,25 Mol Natriumethylat in 100 ml Toluol gegeben. Dazu wird langsam 0,2 Mol Terephthalaldehyddiethylacetal zugetropft und nachgerührt bis zur vollständigen Umsetzung. Nach dem Aufarbeiten und der Reinigung mittels Säulenchromatographie fallen 5 g der Verbindung <u>2A</u> an (Ausbeute: 63 %).

**2B Formyl-zimtsäureethylester**

Diese 35 g (0,12 Mol) der Verbindung 2A werden in 300 ml Dioxan gelöst und in Gegenwart von 2 g Amberlyst 15 bei 20 °C gerührt. Die Aufarbeitung führt zu 23 g (91 %) Formylzimtsäureethylester.

**2C 4-(2'-Hydroxy-1'-oxo-ethyl)-zimtsäureethylester**

Zu einer Reaktionsmischung von 23 g (0,11 Mol) Formylzimtsäureethylester 2B und 3,8 g Paraformaldehyd in 200 ml Ethanol werden 2,7 g (0,011 Mol) 3-Ethyl-benzothiazoliumbromid und Triethylamin zugegeben und unter Rückfluß erhitzt. Nach der Aufarbeitung und Kristallisation fallen weiße Kristalle mit dem Schmelzpunkt von 127 °C an.

Die Spektren entsprechen der erwarteten Verbindung. UV (Ethanol, c = 1 mg/100 ml): $\lambda_{max}$ = 293 nm, E = 1,35
Analog werden hergestellt:

4-(2'-Hydroxy-1'-oxoethyl)-zimtsäurepropylester
4-(2'-Hydroxy-1'-oxoethyl)-zimtsäurehexylester
4-(2'-Hydroxy-1'-oxoethyl)-zimtsäure-(2-ethylhexyl)-ester

**Beispiel 3**

Zu einer Mischung von 35 mMol 9-(4'-Formylbenzyliden)-8-ketotricyclo[5.2.1.0$^{2,6}$]decan(hergestellt aus 8-Ketotricyclo-[5.2.1.0$^{2,6}$]decan und Terephthaldehyd-di ethylacetal in Gegenwart von Natriummethylat und anschließender Acetalspaltung) und 1,2 g Paraformaldehyd in 70 ml Ethanol werden 0,8 g (3,5 mMol) 3-Ethylbenzothiazolium-bromid und Triethylamin gegeben und unter Rückfluß erhitzt. Nach Aufarbeitung analog Beispiel 1 erhält man 9-(4'-(2"-Hydroxy-1"-oxoethyl)-benzyliden)-8-ketotricyclo[5.2.1.0$^{2,6}$]decan. Die Spektren entsprechen der erwarteten Verbindung.

Analog werden hergestellt:

9-(4'-(2"-Hydroxy-1"-oxopropyl)-benzyliden)-8-ketotricyclo-[5.2.1.0$^{2,6}$]decan
9-(4'-(2"-Hydroxy-1"-oxobutyl)-benzyliden)-8-ketotricyclo-[5.2.1.0$^{2,6}$]decan

**Anwendungsbeispiele**

| A | Sonnenschutzcreme (W/O) | | % |
|---|---|---|---|
| A | 3-(4'-2"-Hydroxy-1"-oxoethyl)-benzyliden-campher | (1) | 2,50 |
| | Arlacel 481 | (2) | 6,50 |
| | Arlacel 989 | (2) | 3,50 |
| | Cetiol A | (4) | 2,00 |
| | Paraffinöl dickflüssig (Art.-Nr. 7160) | (1) | 17,00 |
| | Paraffin schüttfähig (Art.-Nr. 7158) | (1) | 3,00 |
| | Miglyol 812 | (3) | 12,00 |
| K | Karion F flüssig (Art.-Nr. 2993) | (1) | 3,00 |
| | Konservierungsmittel | | q.s. |
| | Wasser, demineralisiert | | ad 100,00 |

Herstellung:

Die Phasen A und B auf 75 °C erhitzen. Phase B und in Phase A einrühren. Homogenisieren. Unter Rühren abkühlen.

Bezugsquellen:

(1) E. Merck, Darmstadt
(2) ICI, Essen

(3) Hüls Troisdorf AG, Witten
(4) Fa Henkel, Düsseldorf

| B | Sonnenschutzcreme (W/O) | | % |
|---|---|---|---|
| A | 4-(2'-Hydroxy-1"-oxoethyl)-zimtsäure-ethylester | (1) | 2,50 |
| | Arlacel 481 | (2) | 6,50 |
| | Arlacel 989 | (2) | 3,50 |
| | Cetiol A | (4) | 2,00 |
| | Paraffinöl dickflüssig (Art.-Nr. 7160) | (1) | 17,00 |
| | Paraffin schüttfähig (Art.-Nr. 7158) | (1) | 3,00 |
| | Miglyol 812 | (3) | 12,00 |
| B | Karion F flüssig (Art.-Nr. 2993) | (1) | 3,00 |
| | Konservierungsmittel | | q.s. |
| | Wasser, demineralisiert | | ad 100,00 |

Herstellung:

Die Phasen A und B auf 75 °C erhitzen. Phase B und in Phase A einrühren. Homogenisieren. Unter Rühren abkühlen.

C Untersuchung der Hauthaftung

Es wird eine bestimmte Menge an zu untersuchendem UV-Filter:
A = 3-(4'-(2"-Hydroxy-1"-oxoethyl)-benzyliden)-campher
B = 4-(2'-Hydroxy-1'-oxoethyl)-zimtsäureethylester
auf ein Gelatineblatt aufgetragen und nach angemessener Reaktionszeit wird der nicht gebundene UV-Filter in Ethanol aufgenommen und dessen Menge durch UV-Spektroskopie bestimmt.

Die dabei erhaltenen Ergebnisse sind Tabelle I zu entnehmen:

| UV-Filter | Menge | Reaktionszeit | Menge in ETOH | in Gelatine | Haftung |
|---|---|---|---|---|---|
| | (mg) | | (mg) | (mg) | % |
| A | 2 | 10 min | 0,33 | 1,53 | 83 |
| A | 2 | 30 min | 0,53 | 1,23 | 74 |
| A | 2 | 60 min | 0,29 | 1,58 | 86 |
| A | 2 | 17 hrs | 0,56 | 1,28 | 72 |
| A | 20 | 19 hrs | 16,3 | 1,48 | 18,5 |
| B | 0,5 | 3 Tage | 0,3 | 0,2 | 40 |
| B | 1,0 | 4 Tage | 0,52 | 0,48 | 48 |

| D | Sonnenschutzcreme (W/O) | | |
|---|---|---|---|
| A | 3-(4'-(2"-Hydroxy-1"-oxoethyl)-benzyliden)-campher | (1) | 3.0 % |
| | Eusolex 6300 Art.-Nr.5385 | (1) | 3.0 % |
| | Arlacel 481 | (2) | 6.5 % |
| | Arlacel 989 | (2) | 3.5 % |
| | Cetiol A | (3) | 2.0 % |
| | Paraffinöl dickflüssig Art.-Nr. 7160 | (1) | 17.0 % |
| | Paraffin schüttfähig Art.-Nr. 7158 | (1) | 3.0 % |

(fortgesetzt)

| D | Sonnenschutzcreme (W/O) | | |
|---|---|---|---|
| B | Mygliol 812 | (4) | 12.0 % |
| | Karion F flüssig Art.-Nr. 2993 | (1) | 3.0 % |
| | Konservierungsmittel | (1) | q.s. |
| | Wasser, demineralisiert | | ad 100 % |

Phase A wird auf 75 °C und Phase B auf 80 °C erhitzt. Phase B wird langsam in Phase A eingerührt und homogenisiert.

**Bezugsquellen:**

(1) E. Merck, Darmstadt
(2) ICI, Essen
(3) Henkel, Düsseldorf
(4) Hüls Troisdorf AG, Witten

| E | Sonnenschutzmilch (W/O) | | |
|---|---|---|---|
| A | 3-(4'-(2"-Hydroxy-1"-oxoethyl)-benzyliden)-campher | (1) | 3.0 % |
| | Arlacel 481 | (2) | 3.2 % |
| | Arlacel 989 | (2) | 3.8 % |
| | Paraffinöl dünnflüssig Art.-Nr. 7174 | (1) | 16.0 % |
| | Isopropylmyristat | (3) | 3.5 % |
| | Mygliol 812 | (4) | 3.5 % |
| B | Propandiol-1,2 Art.-Nr. 7478 | (1) | 3.5 % |
| | Magnesiumsulfat-Heptahydrat Art.-Nr. 5882 | (1) | 0.7 % |
| | Konservierungsmittel | (1) | q.s. |
| | Wasser, demineralisiert | | ad 100 % |

Phase A wird auf 75 °C und Phase B auf 80 °C erhitzt. Phase B wird langsam in Phase A eingerührt und homogenisiert.

**Bezugsquellen:**

(1) E. Merck, Darmstadt
(2) ICI, Essen
(3) Henkel, Düsseldorf
(4) Hüls Troisdorf AG, Witten

| F | Sonnenschutzmilch (W/O) | | |
|---|---|---|---|
| A | 3-(4'-(2"-Hydroxy-1"-oxoethyl)-benzyliden)-campher | (1) | 3.0 % |
| | Eusolex 6300 Art.-Nr.5385 | (1) | 3.0 % |
| | Arlacel 481 | (2) | 3.2 % |
| | Arlacel 989 | (2) | 3.8 % |
| | Paraffinöl dünnflüssig Art.-Nr. 7174 | (1) | 16.0 % |
| | Isopropylmyristat | (3) | 3.5 % |
| | Mygliol 812 | (4) | 3.5 % |
| B | Propandiol-1,2 Art.-Nr. 7478 | (1) | 3.5 % |
| | Magnesiumsulfat-Heptahydrat Art.-Nr. 5882 | (1) | 0.7 % |
| | Konservierungsmittel | (1) | q.s. |
| | Wasser, demineralisiert | | ad 100 % |

Phase A wird auf 75 °C und Phase B auf 80 °C erhitzt. Phase B wird langsam in Phase A eingerührt und homogenisiert.

**Bezugsquellen:**

(1) E. Merck, Darmstadt
(2) ICI, Essen
(3) Henkel, Düsseldorf
(4) Hüls Troisdorf AG, Witten

| G | Sonnenschutzcreme (W/O) | | |
|---|---|---|---|
| A | 3-(4'-(2"-Hydroxy-1"-oxoethyl)-benzyliden)-campher | (1) | 3.0 % |
| | Emulgator E 2155 | (2) | 8.0 % |
| | Paraffinöl flüssig Art.-Nr. 7162 | (1) | 12.0 % |
| | Paraffin schüttfähig Art.-Nr. 7158 | (1) | 2.0 % |
| | Isopropylmyristat | (3) | 3.0 % |
| | Mygliol 812 | (4) | 2.0 % |
| B | Propandiol-1,2 Art.-Nr. 7478 | (1) | 4.0 % |
| | Karion F flüssig Art.-Nr. 2993 | (1) | 3.0 % |
| | Konservierungsmittel | (1) | q.s. |
| | Wasser, demineralisiert | | ad 100 % |

Phase A wird auf 75 °C und Phase B auf 80 °C erhitzt. Phase B wird langsam in Phase A eingerührt und homogenisiert.

**Bezugsquellen:**

(1) E. Merck, Darmstadt
(2) ICI, Essen
(3) Henkel, Düsseldorf
(4) Hüls Troisdorf AG, Witten

| H | Sonnenschutzcreme (W/O) | | |
|---|---|---|---|
| A | 3-(4'-(2"-Hydroxy-1"-oxoethyl)-benzyliden)-campher | (1) | 3.0 % |
| | Eusolex 6300 | (1) | 3.0 % |
| | Emulgator E 2155 | (2) | 8.0 % |
| | Paraffinöl flüssig Art.-Nr. 7162 | (1) | 12.0 % |
| | Paraffin schüttfähig Art.-Nr. 7158 | (1) | 2.0 % |
| | Isopropylmyristat | (3) | 3.0 % |
| | Mygliol 812 | (4) | 2.0 % |
| B | Propandiol-1,2 Art.-Nr. 7478 | (1) | 4.0 % |
| | Karion F flüssig Art.-Nr. 2993 | (1) | 3.0 % |
| | Konservierungsmittel | (1) | q.s. |
| | Wasser, demineralisiert | | ad 100 % |

Phase A wird auf 75 °C und Phase B auf 80 °C erhitzt. Phase B wird langsam in Phase A eingerührt und homogenisiert.

**Bezugsquellen:**

(1) E. Merck, Darmstadt
(2) ICI, Essen
(3) Henkel, Düsseldorf
(4) Hüls Troisdorf AG, Witten

| I | Sonnenschutzcreme (W/O) | | |
|---|---|---|---|
| A | 3-(4'-(2"-Hydroxy-1"-oxoethyl)-benzyliden)-campher | (1) | 3.0 % |
| | Arlatone 983 S | (2) | 1.5 % |
| | Arlatone 983 | (2) | 2.2 % |
| | Brij 76 | (2) | 1.5 % |
| | Paraffinöl flüssig Art.-Nr. 7162 | (1) | 5.0 % |
| | Mygliol 812 | (3) | 5.0 % |
| B | Propandiol-1,2 Art.-Nr. 7478 | (1) | 2.5 % |
| | Karion F flüssig Art.-Nr. 2993 | (1) | 2.5 % |
| | Konservierungsmittel | (1) | q.s. |
| | Wasser, demineralisiert | | ad 100 % |

Phase A wird auf 75 °C und Phase B auf 80 °C erhitzt. Phase B wird langsam in Phase A eingerührt und homogenisiert.

**Bezugsquellen:**

(1) E. Merck, Darmstadt
(2) ICI, Essen
(3) Hüls Troisdorf AG, Witten

| J | Sonnenschutzmilch (W/O) | | |
|---|---|---|---|
| A | 3-(4'-(2"-Hydroxy-1"-oxoethyl)-benzyliden)-campher | (1) | 3.0 % |
| | Eusolex 6300 art.-Nr. 5385 | (1) | 3.0 % |
| | Arlatone 983 S | (2) | 1.5 % |
| | Arlatone 983 | (2) | 2.2 % |
| | Brij 76 | (2) | 1.5 % |
| | Paraffinöl flüssig Art.-Nr. 7162 | (1) | 5.0 % |
| | Mygliol 812 | (3) | 5.0 % |
| B | Propandiol-1,2 Art.-Nr. 7478 | (1) | 2.5 % |
| | Karion F flüssig Art.-Nr. 2993 | (1) | 2.5 % |
| | Konservierungsmittel | (1) | q.s. |
| | Wasser, demineralisiert | | ad 100 % |

Phase A wird auf 75 °C und Phase B auf 80 °C erhitzt. Phase B wird langsam in Phase A eingerührt und homogenisiert.

**Bezugsquellen:**

(1) E. Merck, Darmstadt
(2) ICI, Essen
(3) Hüls Troisdorf AG, Witten

**Patentansprüche**

**1.** Verwendung von α-Hydroxyketoalkyl-Derivaten als Lichtschutzfilter für kosmetische Produkte, dadurch gekennzeichnet, daß die α-Hydroxyketoalkylgruppe, gegebenenfalls über einen Spacer, mit einer chromophoren Gruppe, welche in einem Wellenlängenbereich zwischen 280 und 400 nm ein Absorptionsmaximum und ein konjungiertes π-Elektronensystem von mindestens 8 π-Elektronen aufweist, verknüpft ist.

**2.** α-Hydroxyketoalkyl-Derivate der Formel I

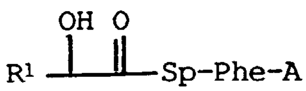 (I)

wobei

R¹ H oder Alkyl mit 1 bis 10 C-Atomen,

Sp $(CH_2)_n$ oder -CH=CH-,

n 0 oder eine ganze Zahl zwischen 1 und 10,

Phe unsubstituiertes oder durch 1 bis 4 Hydroxy-, Alkyl-oder Alkyloxygruppen, worin die Alkylgruppen jeweils 1-10 C-Atome aufweisen, substituiertes Phenylen bedeutet,

A eine Substituentengruppe mit insgesamt bis zu 60 Kohlenstoff-, Schwefel-, Stickstoff- und Sauerstoffatomen in der Grundstruktur bedeutet, welche ein konjungiertes π-Elektronensystem von mindestens 4 π-Elektronen in Konjugation zur Gruppe Phe aufweist.

**3.** α-Hydroxyketoalkyl-Derivate der Formel I nach Anspruch 2, wobei A einen Rest ausgewählt aus den Formeln (a) bis (g) bedeutet:

(a) $-CH=CH-COOR^2$

(b) 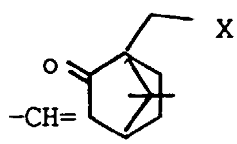

(c) 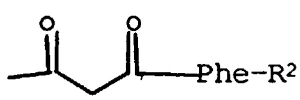

(d) 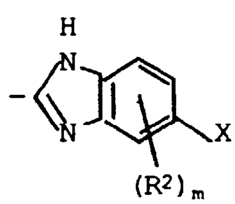

(e) 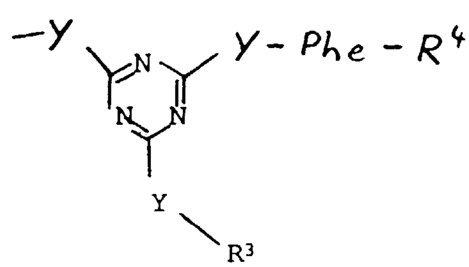

(f) 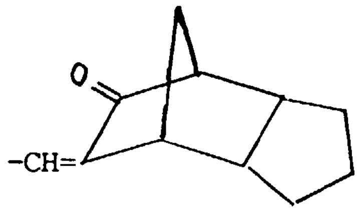

(g) 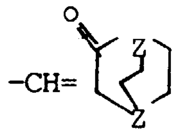

worin jeweils unabhängig voneinander

| | |
|---|---|
| $R^2$ eine für $R^1$ | angegebene Bedeutung besitzt, |
| Phe | die angegebene Bedeutung besitzt, |
| X | H, $R^2$ oder $SO_3H$ bedeutet, |
| m | 0, 1, 2 oder 3 ist, |
| Y | jeweils unabhängig voneinander 0 oder NH ist, |
| Z | jeweils unabhängig voneinander CH oder N |
| $R^3$ | eine der Bedeutungen von $R^1$ besitzt oder Phe-$R^4$ bedeutet, worin $R^4$ einen Rest ausgewählt aus den Formeln (a), (b), (d), (f) und (g) bedeutet, |

bedeuten.

**4.** α-Hydroxyketoalkyl-Derivate nach einem der Ansprüche 2 bis 3, worin
n 0 bedeutet.

**5.** α-Hydroxyketoalkyl-Derivate nach einem der Ansprüche 2 bis 4, worin
$R^1$ H bedeutet.

**6.** α-Hydroxyketoalkyl-Derivate nach einem der Ansprüche 2-5, worin Phe eine unsubstituierte 1,4-Phenylengruppe und $R^1$ H bedeutet.

**7.** Kosmetische Zubereitung, dadurch gekennzeichnet, daß sie eine wirksame Menge mindestens einer Verbindung der Formel I nach Anspruch 2 in einem kosmetisch verträglichen Träger enthält.

**8.** Verwendung der Verbindungen der Formel I nach einem der Ansprüche 2 bis 6 als kosmetisches Produkt.

**9.** Verbindung der Formel I nach einem der Ansprüche 2 bis 6 zur Verwendung als Arzneimittel.

**10.** Pharmazeutische Zubereitung, dadurch gekennzeichnet, daß sie eine wirksame Menge mindestens einer Verbindung der Formel I nach einem der Ansprüche 2 bis 6 in einem physiologisch unbedenklichen Träger oder Exzipienten enthält.

## Claims

1. Use of α-hydroxyketoalkyl derivatives as light protection filters for cosmetic products, characterized in that the α-hydroxyketoalkyl group is linked, if appropriate by a spacer, to a chromophoric group which has an absorption maximum in a wavelength range of between 280 and 400 nm and has a conjugated π-electron system of at least 8 π-electrons.

2. α-Hydroxyketoalkyl derivatives of the formula I

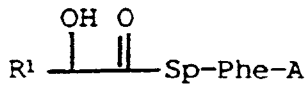 (I)

wherein

$R^1$ is H or alkyl having 1 to 10 C atoms,
Sp is $(CH_2)_n$ or -CH=CH-,
n is 0 or an integer between 1 and 10,
Phe is phenylene which is unsubstituted or substituted by 1 to 4 hydroxyl, alkyl or alkyloxy groups, the alkyl groups in each case having 1-10 C atoms, and
A is a substituent group which has a total of up to 60 carbon, sulfur, nitrogen and oxygen atoms in the basic structure and contains a conjugated π-electron system of at least 4 π-electrons in conjugation with the Phe group.

3. α-Hydroxyketoalkyl derivatives of the formula I according to Claim 2, wherein A is a radical chosen from the formulae (a) to (g):

(a) $-CH=CH-COOR^2$

(b) 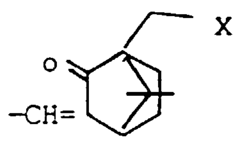

(c) 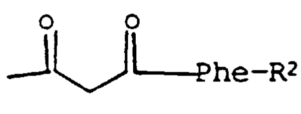

(d) 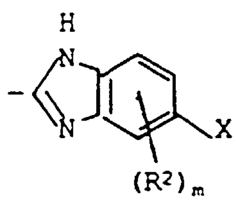

(e) 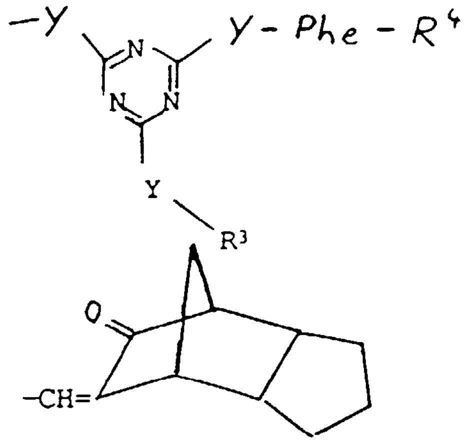

(f)

(g) 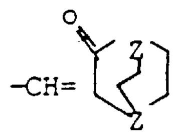

wherein, in each case independently of one another,

- $R^2$ has one of the meanings given for $R^1$,
- Phe has the meaning given,
- X is H, $R^2$ or $SO_3H$,
- m is 0, 1, 2 or 3,
- Y in each case independently of one another is O or NH,
- Z in each case independently of one another is CH or N and
- $R^3$ has one of the meanings of $R^1$ or is Phe-$R^4$, wherein $R^4$ is a radical chosen from the formulae (a), (b), (d), (f) and (g).

**4.** α-Hydroxyketoalkyl derivatives according to one of Claims 2 to 3, wherein n is 0.

**5.** α-Hydroxyketoalkyl derivatives according to one of Claims 2 to 4, wherein
$R^1$ is H.

**6.** α-Hydroxyketoalkyl derivatives according to one of Claims 2-5, wherein Phe is an unsubstituted 1,4-phenylene group and $R^1$ is H.

**7.** Cosmetic formulation, characterized in that it contains an active amount of at least one compound of the formula I according to Claim 2 in a cosmetically compatible carrier.

**8.** Use of the compounds of the formula I according to one of Claims 2 to 6 as a cosmetic product.

**9.** Compound of the formula I according to one of Claims 2 to 6 for use as a medicament.

**10.** Pharmaceutical formulation, characterized in that it comprises an active amount of at least one compound of the formula I according to one of Claims 2 to 6 in a physiologically acceptable carrier or excipient.

**Revendications**

**1.** Utilisation de dérivés d'α-hydroxycétoalkyle comme filtres de protection contre la lumière pour produits cosmétiques, caractérisée en ce que le groupe α-hydroxycétoalkyle est relié, éventuellement par l'intermédiaire d'un groupe d'espacement, à un groupe chromophore qui présente dans un intervalle de longueur d'onde compris entre 280 et 400 nm un maximum d'absorption et un système conjugué d'électrons π comportant au moins 8 électrons π.

**2.** Dérivés d'α-hydroxycétoalkyle de formule I

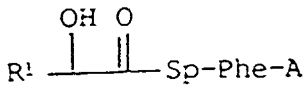

(I)

dans laquelle

$R^1$ représente H ou un alkyle comportant de 1 à 10 atomes de carbone,

Sp représente $(CH_2)_n$ ou -CH=CH- ,

n vaut 0 ou un nombre entier compris entre 1 et 10,

Phe représente un phényle non substitué ou substitué par de 1 à 4 groupes hydroxy, alkyle cu alkyloxy, où les groupes alkyle présentent à chaque fois de 1 à 10 atomes de carbone,

A représente un groupe substituant avec au total jusqu'à 60 atomes de carbone, de soufre, d'azote et d'oxygène dans la structure de base, qui présente un système conjugué d'électrons π d'au moins 4 électrons π en conjugaison avec le groupe Phe.

**3.** Dérivés d'α-hydroxycétoalkyle de formule I selon la revendication 2, dans lesquels A représente un radical choisi parmi les formules (a) à (g):

(a) $-CH{=}CH{-}COOR^2$

(b) 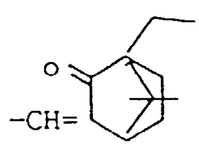

(c) 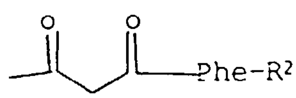

(d) 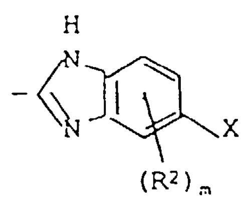

(e) 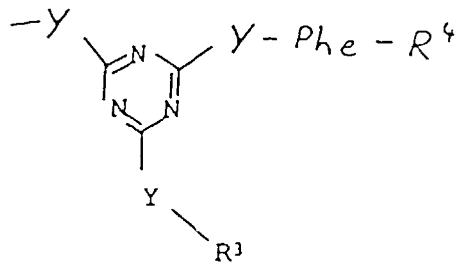

(f) 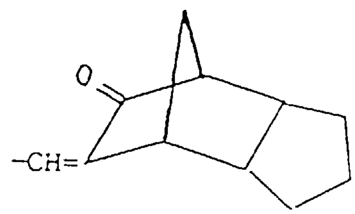

(g) 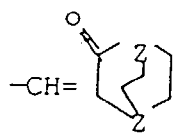

dans lesquelles à chaque fois indépendamment l'un de l'autre

- $R^2$ possède une signification indiquée pour $R^1$,
- Phe possède la signification indiquée,
- X représente H, $R^2$ ou $SO_3H$,
- m vaut 0, 1, 2 ou 3,
- Y représente à chaque fois indépendamment O ou NH,
- Z représente à chaque fois indépendamment CH ou N,
- $R^3$ possède l'une des significations de $R^1$ ou représente Phe-$R^4$, où $R^4$ représente un radical choisi parmi les formules (a), (b), (d), (f) et (g).

**4.** Dérivés d'α-hydroxycétoalkyle selon l'une des revendications 2 à 3, où n vaut 0.

**5.** Dérivés d'α-hydroxycétoalkyle selon l'une des revendications 2 à 4 , où $R^1$ représente H.

**6.** Dérivés d'α-hydroxycétoalkyle selon l'une des revendications 2-5, où Phe représente un groupe 1,4-phénylène non substitué et $R^1$ représente H.

**7.** Préparation cosmétique caractérisée en ce qu'elle contient une quantité efficace d'au moins un composé de formule I selon la revendication 2 dans un support cosmétiquement compatible.

**8.** Application des composés de formule I selon l'une des revendications 2 à 6 comme produit cosmétiques.

**9.** Composé de formule I selon l'une des revendications 2 à 6 aux fins d'application comme médicament.

**10.** Préparation pharmaceutique caractérisée en ce qu'elle contient une quantité efficace d'au moins un composé de formule I selon l'une des revendications 2 à 6 dans un support ou excipient physiologiquement acceptable.